(19) Europäisches Patentamt
European Patent Office
Office européen des brevets

(11) Veröffentlichungsnummer: **0 002 707**
**B1**

(12) **EUROPÄISCHE PATENTSCHRIFT**

(45) Veröffentlichungstag der Patentschrift: **07.04.82**

(21) Anmeldenummer: **78101623.3**

(22) Anmeldetag: **09.12.78**

(51) Int. Cl.³: **C 07 C 97/10,**
C 07 D 295/08,
C 08 F 2/50, C 09 D 11/08

(54) **Neue Aminoalkyl-benzilketale und ihre Verwendung als Initiatoren für die Photopolymerisation ungesättigter Verbindungen.**

(30) Priorität: **22.12.77 CH 15883/77**

(43) Veröffentlichungstag der Anmeldung:
**11.07.79 Patentblatt 79/14**

(45) Bekanntmachung des Hinweises auf die Patenterteilung:
**07.04.82 Patentblatt 82/14**

(84) Benannte Vertragsstaaten:
**FR GB**

(56) Entgegenhaltungen:
**DE - A - 1 769 854**
**DE - A - 2 264 790**
**DE - A - 2 337 813**
**FR - A - 2 295 965**
**US - A - 3 992 276**

(73) Patentinhaber: **CIBA-GEIGY AG**
**Patentabteilung Postfach**
**CH-4002 Basel (CH)**

(72) Erfinder: **Felder, Louis, Dr.**
**Riehenring 5**
**CH-4058 Basel (CH)**
Erfinder: **Kirchmayr, Rudolf, Dr.**
**Ettingerstrasse 9**
**CH-4147 Aesch (CH)**
Erfinder: **Hüsler, Rinaldo, Dr.**
**Schafmattweg 90**
**CH-4102 Binningen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Neue Aminoalkyl-benzilketale und ihre Verwendung als Initiatoren für die Photopolymerisation ungesättigter Verbindungen

Die Erfindung betrifft neue Initiatoren für die Photopolymerisation ungesättigter Verbindungen sowie solche Initiatoren enthaltende Gemische.

Die neuen Photoinitiatoren sind Verbindungen der Formel I,

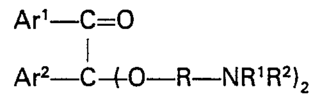

I

worin $Ar^1$ und $Ar^2$ unabhängig voneinander Phenyl oder durch $C_1$—$C_4$ Alkyl, $C_1$—$C_4$ Alkoxy oder Halogen substituiertes Phenyl ist, R $C_2$—$C_4$ Alkylen bedeutet, $R^1$ Wasserstoff, $C_1$—$C_{12}$ Alkyl, Allyl, Benzyl, Cyclohexyl, $C_2$—$C_4$ Hydroxyalkyl oder $C_3$—$C_8$ Alkoxyalkyl und $R^2$ $C_1$—$C_{12}$ Alkyl, Allyl, $C_2$—$C_4$ Hydroxyalkyl, $C_3$—$C_8$ Alkoxyalkyl, Cyclohexyl, Benzyl oder Phenyl bedeuten, oder $R^1$ und $R^2$ zusammen $C_4$—$C_5$ Alkylen oder Oxaalkylen oder eine Gruppe —$CH_2CH_2$—$NR^3$—$CH_2CH_2$- darstellen und $R^3$ Wasserstoff oder $C_1$—$C_4$ Alkyl ist.

$Ar^1$ und $Ar^2$ können beispielsweise Chlorphenyl, Bromphenyl, Tolyl, Xylyl oder Methoxyphenyl sein, vorzugsweise sind sie jedoch Phenyl.

R kann beispielsweise 1,2-Aethylen, 1,3-Propylen, 1,2-Propylen oder 1,2-Butylen sein.

Die Gruppe —$NR^1R^2$ kann beispielsweise Dimethylamino, Diäthylamino, Dibutylamino, Di-2-äthylhexylamino, Didecylamino, Di-dodecyl-amino, Di-2-hydroxyäthyl-amino, Di-2-hydroxypropyl-amino, Methyl-2-hydroxyäthyl-amino, Di-2-methoxyäthyl-amino, Di-2-butoxyäthylamino, Dicyclohexylamino, Aethyl-cyclohexylamino, Butyl-benzylamino, Benzylamino, Cyclohexylamino, Methyl-phenyl-amino, 2-Hydroxyäthyl-phenylamino, Pyrrolidino, Piperidino, Morpholino, Piperazino oder 4-Methylpiperazino.

Die Verbindungen der Formel I stellen Benzil-monoketale mit Aminogruppen im Alkoholrest dar. Benzil-monoketale, die keine Aminogruppen tragen, sind bekannte Verbindungen. Ihre Verwendung als Photoinitiatoren wurde beispielsweise in der US—A—3,715,293 sowie in den DT—A—2 232 365 und 2 337 813 beschrieben.

Die Verbindungen der Formel I sind neue Verbindungen, die ebenfalls als Initiatoren bei der Photopolymerisation von ungesättigten Verbindungen verwendet werden können. Sie zeichnen sich gegenüber den bekannten aminogruppen-freien Verbindungen durch eine erhöhte Wirksamkeit als Photoinitiatoren aus, die sich dadurch äussert, dass zur Härtung der ungesättigten Verbindungen kürzere Belichtungszeiten notwendig sind. Dies entspricht bei der Verwendung in industriellen Anlagen einem grösseren Produktionsausstoss pro Zeiteinheit und pro Belichtungsanlage.

Bevorzugt sind die verbindungen der Formel I, worin $Ar^1$ und $Ar^2$ Phenyl sind, R 1,2-Aethylen ist, $R^1$ und $R^2$ $C_1$—$C_4$ Alkyl sind oder $R^1$ und $R^2$ zusammen —$(CH_2)_5$—, —$CH_2CH_2OCH_2CH_2$— oder —$CH_2CH_2$—$NR^3$—$CH_2CH_2$— darstellen und $R^3$ Wasserstoff oder $CH_3$ ist.

Die Verbindungen der Formel I können allgemein aus den entsprechenden Halogenalkyl-ketalen durch Umsetzung mit Aminen der Formel $R^1$—NH—$R^2$ hergestellt werden:

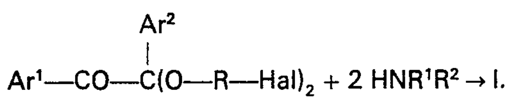

Ein anderes Herstellungsverfahren besteht in der Transacetalisierung der gut zugänglichen Niederalkyl-Ketale mit Aminoalkoholen

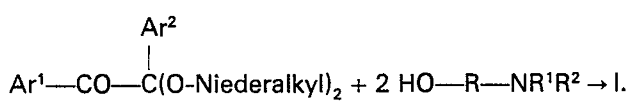

Beispiele für Verbindung der Formel I sind:

Benzil-di-(2-äthylaminoäthyl)-ketal
Benzil-di-(2-dimethylaminoäthyl)-ketal
Benzil-di-(2-morpholinoäthyl)-ketal
Benzil-di-(2-piperidino-äthyl)-ketal
Benzil-di-(2-piperidinoäthyl)-ketal
4,4'-Dichlorbenzil-di-(2-diäthylaminopropyl)-ketal
4,4'-Dichlorbenzil-di-[2-(äthyl-phenyl-amino)-äthyl]-ketal
Benzil-di-[2-(4-methylpiperazino)-äthyl]-ketal
2,2'-Dimethoxybenzil-di-(2-piperidinopropyl)-ketal
4,4'-Dimethylbenzil-di-(2-pyrrolidinoäthyl)-ketal

Die Verbindungen der Formel I können als Initiatoren für die Photopolymerisation von ungesättigten Verbindungen oder Gemischen, die solche Verbindungen enthalten, verwendet werden.

Solche photopolymerisierbare Verbindungen sind beispielsweise ungesättigte Monomere wie Ester von Acryl- oder Methacrylsäure, z.B. Methyl-, Aethyl-, n- oder tert.-Butyl, Isooctyl- oder Hydroxyäthylacrylat, Methyl- oder Aethylmethacrylat, Aethylen-diacrylat.

Neopentyl-diacrylat, Trimethylolpropantrisacrylat, Pentaerythrittetraacrylat oder Pentaerythrittrisacrylat; Acrylnitril, Methacrylnitril, Acrylamid, Methacrylamid, N-substituierte (Meth)-acrylamide; Vinylester wie z.B. Vinyl-acetat, -propionat, -acrylat oder -succinat; sonstige Vinylverbindungen wie Vinyläther, Styrol, Alkylstyrole, Halogenstyrole, Divinylbenzol, Vinylnaphthalin, N-Vinylpyrrolidon, Vinylchlorid oder Vinylidenchlorid; Allylverbindungen wie Diallylphthalat, Diallylmaleat, Triallylisocyanurat, Triallylphosphat oder Aethylenglycol-diallyläther und die Mischungen von solchen ungesättigten Monomeren.

Photopolymerisierbare Verbindungen sind weiterhin ungesättigte Oligomere oder Polymere und deren Mischungen mit ungesättigten Monomeren. Hierzu zählen thermoplastische Harze, die ungesättigte Gruppen wie Fumarsäureester, Allylgruppen oder Acrylat- oder Methacrylatgruppen enthalten. Meist sind diese ungesättigten Gruppen über funktionelle Gruppen an die Hauptkette dieser linearen Polymeren gebunden. Grosse Bedeutung haben Gemische von Oligomeren mit einfach und mehrfach ungesättigten Monomeren. Beispiele für solche Oligomere sind ungesättigte Polyester, ungesättigte Acrylharze und Isocyanat- oder Epoxid- mofifizierte Acrylatoligomere sowie Polyätheracrylat-oligomere. Beispiele für mehrfach ungesättigte Verbindungen sind vor allem die Acrylate von Diolen und Polyolen, z.B. Hexamethylendiacrylat oder Pentaerythrit-tetracrylat. Auch als einfach ungesättigte Monomere werden Acrylate bevorzugt wie z.B. Butylacrylat, Phenylacrylat, Benzylacrylat, 2-Aethyl-hexylacrylat oder 2-Hydroxy-propylacrylat. Man hat es durch Auswahl aus den verschiedenen Vertretern der drei Komponenten in der Hand, die Konsistenz des unpolymerisierten Gemisches sowie die Plastizität des polymerisierten Harzes zu variieren.

Neben diesen Dreikomponenten-Gemischen spielen bei den Polyesterharzen vor allem Zweikomponenten-Gemische eine grosse Rolle. Diese bestehen meist aus einem ungesättigten Polyester und einer Vinylverbindungen. Die ungesättigten Polyester sind oligomere Veresterungsprodukte mindestens einer ungesättigten Dicarbonsäure wie z.B. Malein-, Fumar- oder Citraconsäure, und meist mindestens einer gesättigten Dicarbonsäure, wie z.B. Phthalsäure, Bernsteinsäure, Sebazinsäure oder Isophthalsäure, mit Glykolen wie z.B. Aethylenglykol, Propandiol-1,2, Di- oder Triäthylenglykol oder Tetramethylenglykol, wobei zur Modifizierung meist auch Monocarbonsäuren und Monoalkohole mitverwendet werden. Diese ungesättigten Polyester werden üblicherweise in einer Vinyl- oder Allylverbindung gelöst, bevorzugt wird hierfür Styrol verwendet.

Photopolymerisierbare Gemische, wie sie für die verschiedenen Zwecke verwendet werden, enthalten meist ausser den photopolymerisierbaren Verbindungen und dem Photoinitiator eine Reihe sonstiger Zusätze. So ist es vielfach üblich, thermische Inhibitoren zuzusetzen, die vor allem während der Herstellung der Gemische durch Mischen der Komponenten vor einer vorzeitigen Polymerisation schützen sollen. Hierzu werden beispielsweise Hydrochinon, Hydrochinonderivate, p-Methoxyphenyl oder β-Naphthole verwendet. Weiter können geringe Mengen von UV-Absorbern zugesetzt werden wie z.B. solche vom Benztriazol- oder Benzophenontyp.

Zur Erhöhung der Dunkellagerstabilität können Kupferverbindungen wie Kupfernaphthenat, -stearat, oder -octoat, Phosphorverbindungen wie Triphenylphosphin, Tributylphosphin, Triäthylphosphit, Triphenylphosphit oder Tribenzylphosphat, quartäre Ammoniumverbindungen wie Tetramethylammoniumchlorid oder Trimethyl-benzylammoniumchlorid oder Hydroxylaminderivate wie z.B. N-Diäthylhydroxylamin zugesetzt werden. Weiterhin können die photopolymerisierbaren Gemische Kettenübertragungsmittel wie z.B. N-Methyldiäthanolamin, Triäthanolamin oder Cyclohexen enthalten.

Um die inhibierende Wirkung des Luftsauerstoffs auszuschliessen setzt man photohärtbaren Gemischen häufig Paraffin oder ähnliche wachsartige Stoffe zu. Diese schwimmen bei Beginn der Polymerisation wegen mangelnder Löslichkeit im Polymeren aus und bilden eine transparente Oberflächenschicht, die den Zutritt von Luft verhindert. Auch durch Einführung von autoxydablen Gruppen, beispielsweise Allylgruppen, in das zu härtende Harz kann der Luftsauerstoff desaktiviert werden.

Photopolymerisierbare Gemische enthalten weiterhin—je nach Verwendungszeck—Füllstoffe wie Kieselsäure, Talkum oder Gips, Pigmente, Farbstoffe, Fasern, Thixotropiemittel oder Verlaufhilfsmittel.

Die erfindungsgemässen Photoinitiatoren können auch in Kombination mit radikalischen Initiatoren wie z.B. Peroxiden, Hydroperoxiden, Keton-peroxiden oder Percarbonsäureestern verwendet werden.

Ferner können auch Kombinationen mit bekannten Photoinitiatoren, wie Benzoinäthern, Dialkoxyacetophenonen oder Benzilketalen, oder Kombinationen mit aromatischen Ketonen verwendet werden. Beispiele für solche Ketone sind Benzophenon, substituierte Benzophenonderivate, Michler's Keton, Anthrachinon und Anthrachinonderivate, sowie Thioxanthon und dessen Derivate.

Grosse Bedeutung hat die Photohärtung für Druckfarben, da die Trocknungszeit des Bindemittels ein massgeblicher Faktor für die Produktionsgeschwindikeit graphischer Erzeugnisse ist und in der Grössenordnung von Bruchteilen von Sekunden liegen soll. Gut geeignet sind die erfindungsgemässen Initiatoren auch für photohärtbare Gemische zur Herstellung von Druckplatten. Hierbei werden meist Gemische von löslichen linearen Polyamiden mit photopolymerisierbaren Monomeren, beispielsweise

Acrylamide, und einem Photoinitiator verwendet. Filme oder Platten aus diesen Gemischen werden über das Negativ (oder Positiv) der Druckvorlage belichtet und die ungehärteten Anteile anschliessend mit einem Lösungsmittel eluiert.

Ein weiteres Einsatzgebiet der UV-Härtung ist die Metallbeschichtung, beispielsweise bei der Lackierung von Blechen für Tuben, Dosen oder Flaschenverschlüssen, sowie die UV-Härtung von Kunststoffbeschichtungen, beispielsweise von Fussboden- oder Wandbelägen auf PVC-Basis.

Beispiele für die UV-Härtung von Papierbeschichtungen sind die farblose Lackierung von Ettiketten, Schallplatten-Hüllen oder Buchumschlägen.

Die Photoinitiatoren werden für die angeführten Anwendungsgebiete zweckmässig in Mengen von 0,1 bis 20 Gew.%, vorzugsweise etwa 0,5 bis 5 Gew.-%, bezogen auf das photopolymerisierbare Gemisch, angewendet. Unter Gemisch ist hierbei das Gemisch aus der photopolymerisierbaren ungesättigten Verbindung, dem Photoinitiator und den sonstigen Füll- und Zusatzstoffen gemeint wie es in der jeweiligen Anwendung verwendet wird.

Der Zusatz der Photoinitiatoren zu den photopolymerisierbaren Verbindungen geschieht im allgemeinen durch einfaches Einrühren, da die meisten dieser Verbindungen flüssig oder gut löslich sind. Meist kommt es zu einer Lösung der erfindungsgemässen Initiatoren, wodurch deren gleichmässige Verteilung sowie die Transparenz der Polymerisate gewährleitstet ist.

Die Polymerisation erfolgt nach den bekannten Methoden der Photopolymerisation durch Bestrahlung mit Licht, das reich an kurzwelliger Strahlung ist. Als Lichtquellen sind z.B. Quecksilbermitteldruck-, -hochdruck- und -niederdruckstrahler, sowie superaktinische Leuchtstoffröhren geeignet, deren Emissionsmaxima im Bereich zwischen 250 und 400 nm liegen.

Die folgenden Beispiele beschreiben die Herstellung und Verwendung von Verbindungen der Formel I noch näher. Hierin bedeuten Teile Gewichtsteile und Prozente Gewichtsprozente.

## Beispiel 1

*Benzil-di-(2-morpholino-äthyl)-ketal*

(2,2-Di-(2-morpholino-äthoxy)-1,2-diphenyl-äthanon)

35 g (0,1 Mol) Benzil-di-(2-chloräthyl)-ketal werden mit 52 g (0,6 Mol) Morpholin, unter Rühren, 6 Stunden bei 110°C gehalten. Nach dem Abkühlen wird das Produkt in Aether aufgenommen. Die Aetherschicht wird mit Wasser gewaschen, mit $Na_2SO_4$ getrocknet und eingeengt. Das als Oel anfallende Produkt wird im Hochvakuum bei 50—60°C getrocknet.

Ausbeute: 41 g (90%)

$C_{26}H_{34}N_2O_5$

| | | | |
|---|---|---|---|
| Berechnet: | C 68,70% | H 7,54% | N 6,16% |
| Gefunden: | C 68,7% | H 7,7% | N 6,1% |

## Beispiel 2

Eine Harzmischung aus 80 Teilen Plex 6616® (Acrylatharz der Firma Röhm, Darmstadt), 20 Teilen Trimethylolpropan- tris-acrylat und 2 Teilen des in Beispiel 1 beschriebenen Photoinitiators wird mit einem Filmziehgerät in einer Dicke von 40 $\mu$m auf Glasplatten aufgezogen. Diese Filme werden ca. 20 Sekunden abgelüftet und anschliessend mit einem Hg-Mitteldruckbrenner (Hanovia-Gerät, Modell 45080) bestrahlt. Dabei werden die Proben auf einem Transportband mit einer solchen Geschwindigkeit unter der UV-Lampe vorbeibewegt, dass sich eine effektive Belichtungszeit von 0,16 Sekunden pro Durchlauf ergibt.

Unter diesen Bedingungen sind 7 Durchläufe nötig, um einen klebfreien Film zu erhalten. Die Pendelhärte nach König des Filmes beträgt nach 7 Durchläfen 124, nach 9 Durchläufen 127 und nach 11 Durchläufen 139.

## Beispiel 3

*Benzil-di-(2-diäthylamino-äthyl)-ketal*

(2,2-Di-(2-diäthylamino-äthoxy)-1,2-diphenyl-äthanon)

20 g (0,057 Mol) Benzil-di-(2-chloräthyl)-ketal werden mit 150 g (2,05 Mol) Diäthylamin 24 Stunden bei 140°C im Druckautoklaven gerührt. Nach dem Abkühlen wird das Reaktionsgemisch in Aether aufgenommen und mit Wasser ausgeschüttelt. Die Aetherschicht wird mit $Na_2SO_4$ getrocknet und eingeengt. Das als Oel anfallende Produkt wird im Hochvakuum getrocknet.

$C_{26}H_{38}N_2O_3$

| | | | |
|---|---|---|---|
| Berechnet: | C 73,20% | H 8,95% | N 6,57% |
| Gefunden: | C 73,20% | H 9,1% | N 6,3%. |

## Beispiel 4

*Benzil-di-(2-piperidino-äthyl)-ketal*

(2,2-Di-(2-piperidino-äthoxy)-1,2-diphenyl-äthanon)

35 g (0,1 Mol) Benzil-di-(2-chloräthyl)-ketal werden mit 77 g (0,9 Mol) Piperidin unter Rühren 7 Stunden bei 105°C gehalten. Nach dem Abkühlen wird mit Aether verdünnt und das Piperidinhydrochlorid abfiltriert. Der Aether wird abdestilliert und das als Oel anfallende Produkt wird im Hochvakuum getrocknet.

| | | | |
|---|---|---|---|
| $C_{28}H_{38}N_2O_3$ | | | |
| Berechnet: | C 74,63% | H 8,50% | N 6,22% |
| Gefunden: | C 74,6% | H 9,0% | N 6,6% |

Beispiel 5

*Benzil-di-(2-(4-methylpiperazino)äthyl)-ketal*

(2,2-Di-(2-(4-methylpiperazino)äthoxa)-1,2-diphenyl-äthanon)

23,6 g (0,067 Mol) Benzil-di-(2-chloräthyl)-ketal werden mit 120 g (1,2 Mol) N-Methylpiperazin unter Rühren 3 Stunden bei 95°C gehalten. Nach dem Abkühlen wird 250 ml Toluol zugegeben und auf Waser gegossen. Die Wasserschicht wird mit Toluol extrahiert und die Toluolschicht anschliessend mit Wasser neutral gewaschen. Die Toluolschicht wird mit $Na_2SO_4$ getrocknet, eingeengt und im Hochvakuum getrocknet.

| | | | |
|---|---|---|---|
| $C_{28}H_{40}N_3O_3$ | | | |
| Berechnet: | C 69,97% | H 8,39% | N 11,66% |
| Gefunden: | C 70,0% | H 8,5% | N 11,1%. |

Beispiel 6

Eine Harzmischung aus 70 Teilen Ebecryl 593® (Polyester-Acrylat der Firma UCB, Belgien) 30 Teilen Trimethylolpropantriacrylat, 0,5 Teilen Byk 300® (Verlaufhilfsmittel der Firma Byk-Mallinckrodt, Deutschland) und 3 Teilen Photoinitiator wird mit einem Aufziehrahmen in einer Schichtdicke von 30—40 $\mu$ auf Glasplatten aufgetragen. Diese werden nach kurzer Abluftzeit in einem UV-Laborgerät (Modell PPG, QC-Prozessor) durch Belichtung mit einer UV-Lampe von 80 Watt/cm ausgehärtet. Nach der UV-Härtung wird 1/2 Stunde im Normklima gelagert und anschliessend die Pendelhärte nach König bestimmt. In der folgenden Tabelle werden die gemessenen Pendelhärten in Abhängigkeit von der Transportgeschwindigkeit im Bestrahlungsgerät dargestellt:

| Photoinitiator | Pendelhärte in sec. bei Transportgeschwindigkeit von | | |
|---|---|---|---|
| | 10 m/min | 25 m/min | 75 m/min |
| Benzildimethylketal (Vergleichssubstanz) | 151 | 143 | 92 |
| Verbindung gemäss Beispiel 3 | 158 | 155 | 114 |
| Verbindung gemäss Beispiel 4 | 152 | 145 | 120 |

**Patentansprüche**

1. Verbindungen der Formel I

$$\begin{array}{l} Ar^1—C=O \\ \quad\quad | \\ Ar^2—C—(O—R—NR^1R^2)_2 \end{array} \qquad I$$

worin $Ar^1$ und $Ar^2$ unabhängig voneinander Phenyl oder durch $C_1$—$C_4$ Alkyl, $C_1$—$C_4$ Alkoxy oder Halogen substituiertes Phenyl ist, R $C_2$—$C_4$ Alkylen bedeutet, $R^1$ Wasserstoff, $C_1$—$C_{12}$ Alkyl, Allyl, Benzyl, Cyclohexyl, $C_2$—$C_4$ Hydroxyalkyl oder $C_3$—$C_8$ Alkoxyalkyl und $R^2$ $C_1$—$C_{12}$ Alkyl, Allyl, $C_2$—$C_4$ Hydroxyalkyl, $C_3$—$C_8$ Alkoxyalkyl, Cyclohexyl, Benzyl oder Phenyl bedeuten, oder $R^1$ und $R^2$ zusammen, $C_4$—$C_5$ Alkylen oder Oxaalkylen oder eine Gruppe $—CH_2CH_2—NR^3—CH_2CH_2—$ darstellen und $R^3$ Wasserstoff oder $C_1$—$C_4$ Alkyl ist.

2. Verbindungen der Formel I gemäss Anspruch 1, worin $Ar^1$ und $Ar^2$ Phenyl sind, R 1,2-Aethylen ist und $R^1$ und $R^2$ $C_1$—$C_4$ Alkyl, oder $R^1$ und $R^2$ zusammen $—(CH_2)_5—$, $—CH_2CH_2OCH_2CH_2—$ oder $—CH_2CH_2—NR^3—CH_2CH_2—$ darstellen, worin $R^3$ Wasserstoff oder $CH_3$ ist.

3. Die Verbindung der Formel

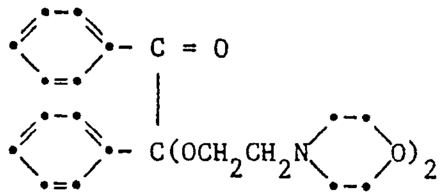

gemäss Anspruch 1.

4. Die Verbindung der Formel

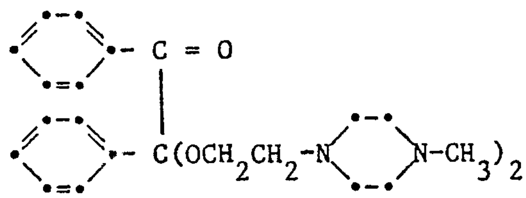

gemäss Anspruch 1.

5. Verwendung von Verbindungen der Formel I gemäss Anspruch 1 als Initiatoren für die Photopolymerisation ungesättigter Verbindungen.

6. Photopolymerisierbares Gemisch aus mindestens einer ungesättigten Verbindung, 0,1 bis 20, vorzugsweise 0,5 bis 5 Gew.-% eines Photopolymerisationsinitiators und gegebenenfalls Zusatzstoffen, dadurch gekennzeichnet, dass es als Photopolymerisationsinitiator eine Verbindung gemäss Formel I des Anspruches 1 enthält.

**Revendications**

1. Composés répondant à la formule I

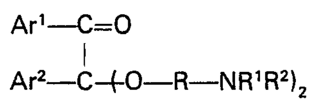

I

dans laquelle $Ar^1$ et $Ar^2$ représentent chacun, indépendamment l'un de l'autre, un radical phényle éventuellement porteur d'un alkyle en $C_1$—$C_4$, d'un alcoxy en $C_1$—$C_4$ ou d'un halogène, R représente un alkylène en $C_2$—$C_4$, $R^1$ représente l'hydrogène, un alkyle en $C_1$—$C_{12}$, un allyle, un benzyle, un cyclohexyle, un hydroxyalkyle en $C_2$—$C_4$ ou un alcoxy-alkyle en $C_3$—$C_8$, et $R^2$ représente un alkyle en $C_1$—$C_{12}$, un allyle, un hydroxy-alkyle en $C_2$—$C_4$, un alcoxy-alkyle en $C_3$—$C_8$, un cyclo-hexyle, un benzyle ou un phényle, ou $R^1$ et $R^2$ forment ensemble un alkylène en $C_4$ ou $C_5$, un oxaalkylène ou un radical —$CH_2CH_2$—$NR^3$—$CH_2CH_2$— dans lequel $R^3$ désigne l'hydrogène ou un alkyle en $C_1$—$C_4$.

2. Composés de formule I selon la revendication 1 dans lesquels $Ar^1$ et $Ar^2$ représentent chacun un radical phényle, R représente un radical éthylène-1,2 et $R^1$ et $R^2$ représentent chacun un alkyle en $C_1$—$C_4$, ou $R^1$ et $R^2$ forment ensemble un radical —$(CH_2)_5$—, —$CH_2CH_2OCH_2CH_2$— ou —$CH_2CH_2$—$NR^3$—$CH_2CH_2$—, le symbole $R^3$ désignant l'hydrogène ou $CH_3$.

3. Composé selon la revendication 1, en l'espèce celui qui répond à la formule

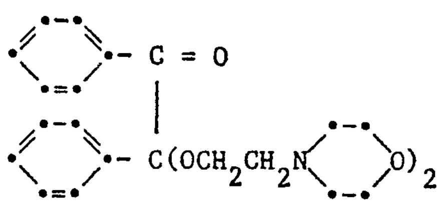

4. Composé selon la revendication 1, en l'espèce celui qui répond à la formule

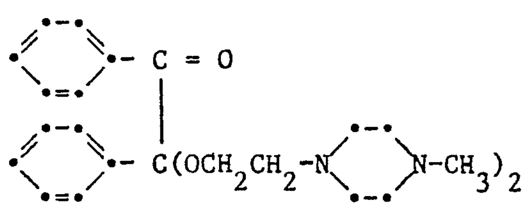

5. Application de composés de formule I selon la revendication 1 comme amorceurs pour la photopolymérisation de composés insaturés.

6. Mélange photopolymérisable constitué d'au moins un composé insaturé, de 0,1 à 20% en poids, de préférence de 0,5 à 5% en poids, d'un amorceur de photopolymérisation et, éventuellement, d'additifs, mélange caractérisé en ce qu'il contient, comme amorceur de photopolymérisation, un composé de formule I selon la revendication 1.

## Claims

1. A compound of the formula I

$$\begin{array}{l} Ar^1—C=O \\ \qquad\;| \\ Ar^2—C—(O—R—NR^1R^2)_2 \end{array} \qquad I$$

wherein $Ar^1$ and $Ar^2$, each independently of the other, is phenyl or phenyl which is substituted by $C_1$—$C_4$ alkyl, $C_1$—$C_4$ alkoxy or halogen, R is $C_2$—$C_4$ alkylene, $R^1$ is hydrogen, $C_1$—$C_{12}$ alkyl, allyl, benzyl, cyclohexyl, $C_2$—$C_4$ hydroxyalkyl or $C_3$—$C_8$ alkoxyalkyl, and $R^2$ is $C_1$—$C_{12}$ alkyl, allyl, $C_2$—$C_4$-hydroxyalkyl, $C_3$—$C_8$ alkoxyalkyl, cyclohexyl, benzyl or phenyl, or $R^1$ and $R^2$ together are $C_4$—$C_5$ alkylene or oxaalkylene or a group $—CH_2CH_2—NR^3—CH_2CH_2—$, and $R^3$ is hydrogen or $C_1$—$C_4$ alkyl.

2. A compound of the formula I according to claim 1 wherein $Ar^1$ and $Ar^2$ are phenyl, R is 1,2-ethylene and each of $R^1$ and $R^2$ is $C_1$—$C_4$ alkyl, or $R^1$ and $R^2$ together are $—(CH_2)_5—$, $—CH_2CH_2OCH_2CH_2—$ or $—CH_2CH_2—NR^3—CH_2CH_2$, wherein $R^3$ is hydrogen or $CH_3$.

3. The compound of the formula

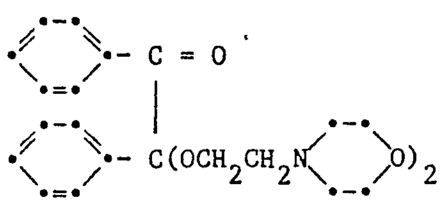

according to claim 1.

4. The compound of the formula

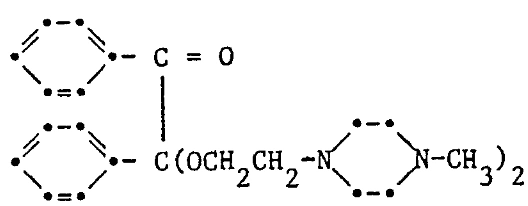

according to claim 1.

5. Use of a compound of the formula I according to claim 1 as initiator for the photopolymerisation of unsaturated compounds.

6. A photopolymerisable mixture consisting of at least one unsaturated compound, 0.1 to 20% by weight, preferably 0.5 to 5% by weight, of a photoinitiator, and optionally additives, wherein said photoinitiator is a compound of the formula I according to claim 1.